(19) Europäisches Patentamt

European Patent Office

Office européen des brevets

(11) EP 0 694 531 B1

## (12) EUROPÄISCHE PATENTSCHRIFT

(45) Veröffentlichungstag und Bekanntmachung des Hinweises auf die Patenterteilung:
**16.09.1998 Patentblatt 1998/38**

(51) Int. Cl.$^6$: **C07C 275/60**, C08G 18/81, C09D 175/16

(21) Anmeldenummer: **95111163.2**

(22) Anmeldetag: **17.07.1995**

(54) **Verfahren zur Herstellung von wasserverdünnbaren Urethanharzen und deren Verwendung**

Process for the preparation of water-thinnable urethane resins and their use

Procédé de préparation de résines d'uréthane diluables à l'eau et leur utilisation

(84) Benannte Vertragsstaaten:
**BE CH DE DK FR GB IT LI NL SE**
Benannte Erstreckungsstaaten:
**LT SI**

(30) Priorität: **27.07.1994 AT 1481/94**

(43) Veröffentlichungstag der Anmeldung:
**31.01.1996 Patentblatt 1996/05**

(73) Patentinhaber:
**Vianova Resins Aktiengesellschaft
8402 Werndorf (AT)**

(72) Erfinder:
• **Gerlitz, Martin, Dr.
A-8055 Graz (AT)**
• **Awad, Rami-Raimund, Dr.
A-8042 Graz (AT)**
• **Fraydl, Thomas
A-8041 Graz (AT)**

(56) Entgegenhaltungen:
**EP-A- 0 453 838**

• **CHEMICAL ABSTRACTS, vol. 79, no. 16, 22.Oktober 1973 Columbus, Ohio, US; abstract no. 93572g, Seite 75; & JP-A-07 343 729 (KANSAI PAINT CO.) 23.Juni 1973**

**Beschreibung**

Die Erfindung betrifft ein Verfahren zur Herstellung von wasserverdünnbaren Urethanharzen und deren Verwendung als Bindemittel für durch radikalische Polymerisation vernetzbare Lacke, die in der Anwendungsform frei von Monomeren und organischen Lösemitteln vorliegen, emissionsfrei härtbar sind und als Lackfilm einen hohen Vernetzungsgrad aufweisen.

In Wasser lösliche oder dispergierbare Polyurethane, die Carboxylgruppen in Form von Struktureinheiten der Dimethylolpropionsäure enthalten und die sich als Beschichtungsmittel für beliebige Substrate eignen, sind beispielsweise aus der EP 0269 972 A2 bekannt.

Die Herstellung ähnlich aufgebauter Oligourethane, welche überdies durch radikalische Polymerisation härtbar sind, ist in der EP 0453 838 A2 beschrieben. Dabei wird ein Gemisch aus (Meth)acryloylgruppen aufweisenden Alkoholen und Dimethylolpropionsäure in Gegenwart eines organischen Lösemittels, wie Butylacetat, mit Diisocyanaten umgesetzt und das Reaktionsprodukt mittels Ammoniak in die wasserverdünnbare Form übergeführt.

Derartige Oligourethane weisen wesentliche Nachteile auf. Vor dem Dispergieren in Wasser liegen die Produkte als 60 bis 95 %ige Lösungen in organischen Lösemitteln vor, die nur zum Teil entfernt werden können, da die Viskosität der Harze mit steigendem Feststoffgehalt rasch zunimmt. Auch wird Ammoniak als Neutralisationsmittel für wasserverdünnbare Lacke immer häufiger abgelehnt.

Es wurde nun gefunden, daß durch eine spezielle Auswahl der Rohstoffe und vor allem durch spezielle Verfahrensschritte die Herstellung von wasserverdünnbaren Urethanharzen möglich ist, die in der Anwendungsform frei von Monomeren und organischen Lösemitteln vorliegen und emissionsfrei härtbar sind.

Die Erfindung betrifft demgemäß ein Verfahren zur Herstellung von wasserverdünnbaren Urethanharzen, welches dadurch gekennzeichnet ist, daß man

| | |
|---|---|
| (A) 1,0 Mol | Hexamethylendiisocyanat, dessen NCO-Gruppen nach der Reaktion mit (Meth)acryloylgruppen enthaltenden Alkoholen, welche als eine oder zwei freie Hydroxylgruppe(n) aufweisende Ester der (Meth)acrylsäure mit zwei- bis vierwertigen Alkoholen bzw. mit Mono- oder Diglycidylverbindungen zu definieren sind, und gegebenenfalls mit aliphatischen Monoalkoholen, deren Alkylreste sechs bis vierzehn C-Atome aufweisen, zu insgesamt 50 Mol-% als Urethangruppen vorliegen, |
| | mit |
| (B) 0,25 bis 0,45 Mol | 2,2-Bis-(hydroxymethyl)-propionsäure bei 70 bis 90°C bis zur vollständigen Reaktion der Hydroxylgruppen umsetzt, anschließend das so erhaltene Zwischenprodukt (AB) mit |
| (C) 0,2 bis 0,45 Mol | eines aliphatischen und/oder cycloaliphatischen Diisocyanats, dessen NCO-Gruppen nach der Reaktion mit (Meth)acryloylgruppen enthaltenden Alkoholen, welche als eine einzige freie Hydroxylgruppe aufweisende Ester der (Meth)acrylsäure mit zwei- bis vierwertigen Alkoholen bzw. mit Mono- oder Diglycidylverbindungen zu definieren sind, und gegebenenfalls mit aliphatischen Monoalkoholen, deren Alkylreste sechs bis vierzehn C-Atome aufweisen, zu insgesamt 50 Mol-% als Urethangruppen vorliegen, |

bei 100 bis 110°C bis zur vollständigen Reaktion der restlichen freien Isocyanatgruppen zu Allophanatgruppen umsetzt, wobei die Molverhältnisse der Komponenten (A) bis (C) so gewählt werden, daß sich die Äquivalente der in den ursprünglichen Rohstoffen für die Komponenten (A) bis (C) enthaltenen Isocyanatgruppen und Hydroxylgruppen wie 1,1 : 1 bis 1,45 : 1 verhalten und das Reaktionsprodukt Carboxylgruppen entsprechend einer Säurezahl von 25 bis 50 mg KOH/g aufweist, die zu mindestens 45 % mit einem (D) Alkalihydroxid, gegebenenfalls als Gemisch mit einem (E) aliphatischen oder cycloaliphatischen Diisocyanat, dessen NCO-Gruppen nach der Reaktion zu je 50 Mol-% mit (Meth)acryloylgruppen enthaltenden Alkoholen, welche wie bei Komponente (C) als eine einzige freie Hydroxylgruppe aufweisende Ester der (Meth)acrylsäure mit zwei- bis vierwertigen Alkoholen bzw. mit Mono- oder Diglycidylverbindungen zu definieren sind, sowie mit N,N-Dialkylalkanolaminen als Urethangruppen vorliegen, neutralisiert werden, mit der Maßgabe, daß das Endprodukt ein Doppelbindungsäquivalent (Mol-Zahl der ethylenischen Doppelbindungen pro 1000 g Harz als Feststoff) von 1,5 bis 3,5 mMol/g aufweist.

Die Erfindung betrifft weiters die Verwendung der erfindungsgemäß hergestellten Urethanharze als Bindemittel für durch radikalische Polymerisation vernetzbare wasserverdünnbare Lacke.

Die Komponente (A) besteht aus Hexamethylendiisocyanat, dessen NCO-Gruppen nach der Reaktion mit (Meth)acryloylgruppen enthaltenden Alkoholen und gegebenenfalls mit aliphatischen Monoalkoholen zu insgesamt 50 Mol-% als Urethangruppen vorliegen.

Bei den (Meth)acryloylgruppen enthaltenden Alkoholen handelt es sich um eine oder zwei freie Hydroxylgruppe(n) aufweisende Ester der (Meth)acrylsäure mit zwei- bis vierwertigen Alkoholen bzw. mit Mono- oder Diglycidylverbindungen.

Die in der Komponente (A) zur Urethanisierung von NCO-Gruppen gegebenenfalls verwendeten aliphatischen Monoalkohole weisen Alkylreste mit sechs bis vierzehn C-Atomen auf. Der Anteil dieser Monoalkohole, bezogen auf die gesamte Menge an Hydroxylgruppen, die zur Umsetzung der Isocyanatgruppen zur Verfügung stehen, beträgt maximal 40 Mol-%.

Als Komponente (B) wird 2,2-Bis- (hydroxymethyl)-propionsäure (Dimethylolpropionsäure, DMPS) eingesetzt.

Die Komponente (C) entspricht in ihrer Zusammensetzung weitgehend der Komponente (A). Im Unterschied zu dieser können als Rohstoffe neben dem bevorzugten Hexamethylendiisocyanat auch ein anderes aliphatisches Diisocyanat und/oder ein cycloaliphatisches Diisocyanat, vorzugsweise Isophorondiisocyanat, verwendet werden. Die (Meth)acryloylgruppen enthaltenden Alkohole dürfen nur eine einzige freie Hydroxylgruppe aufweisen.

Die Komponenten (A) und (C) werden in bekannter Weise hergestellt, wobei die Diisocyanate vorgelegt und die Alkohole innerhalb eines Zeitraums von 30 Minuten bis 3 Stunden unter Berücksichtigung der auftretenden Exothermie so zugegeben werden, daß eine Reaktionstemperatur von 40 bis 55°C nicht überschritten wird. Das Reaktionsgemisch wird solange gerührt, bis der theoretische NCO-Gehalt erreicht ist.

Nur bei einer möglichst quantitativen Umsetzung der Diisocyanate zu den entsprechenden "Monourethanen" werden die erforderlichen niedermolekularen und somit ausreichend niedrigviskosen Zwischenprodukte erhalten.

Als Komponente (D) wird neben NaOH oder KOH bevorzugt Lithiumhydroxid verwendet.

Die Komponente (E) besteht aus einem aliphatischen oder cycloaliphatischen Diisocyanat, dessen Isocyanatgruppen nach der Reaktion zu je 50 Mol-% mit (Meth)acryloylgruppen enthaltenden Alkoholen sowie mit N,N -Dialkylalkanolaminen, wie Dimethyl- oder Diethylethanolamin, als Urethangruppen vorliegen.

Zur Herstellung der Komponente (E) wird eine Komponente (C) mit äquimolaren Mengen des Dialkylalkanolamins umgesetzt, wobei die Reaktionstemperatur von max. 50°C nicht überschritten werden soll. Das Reaktionsgemisch wird so lange gerührt, bis keine freien NCO-Gruppen nachzuweisen sind.

Zur Durchführung des erfindungsgemäßen Verfahrens wird 1,0 Mol der Komponente (A) mit 0,25 bis 0,45 Mol 2,2-Bis-(hydroxymethyl)-propionsäure (Komponente B) bei 70 bis 90°C bis zur vollständigen Reaktion der Hydroxylgruppen umgesetzt. Das so erhaltene Zwischenprodukt (AB) wird anschließend mit 0,2 bis 0,45 Mol der Komponente (C) bei 100 bis 110°C bis zur vollständigen Reaktion der restlichen freien Isocyanatgruppen zu Allophanatgruppen umgesetzt. Die Molverhältnisse der Komponenten (A) bis (C) werden so gewählt, daß sich die Äquivalente der in den Rohstoffen für die Komponenten (A) bis (C) ursprünglich vorhandenen Isocyanatgruppen und Hydroxylgruppen wie 1,1 : 1 bis 1,45 : 1 verhalten.

Entsprechend einer Säurezahl von 25 bis 50 mg KOH/g enthält das Reaktionsprodukt Carboxylgruppen, die zu mindestens 45 % mit der Komponente (D), gegebenenfalls im Gemisch mit der Komponente (E), wobei deren Anteil bis zu 50 Äquivalent-% betragen kann, neutralisiert werden.

Das Endprodukt weist ein Doppelbindungsäquivalent (Mol-Zahl der ethylenischen Doppelbindungen pro 1000 g Harz als Feststoff) von 1,5 bis 3,5 mMol/g auf.

Die Auswahl spezifischer Rohstoffe und der eingeschlagene Syntheseweg ermöglichen es somit, niedrigviskose Urethanharze herzustellen. Sie werden als Bindemittel für durch radikalische Polymerisation, insbesonders mittels UV-Bestrahlung, vernetzbare Lacke verwendet, die in der Anwendungsform frei von Monomeren und organischen Lösemitteln vorliegen, emissionsfrei härtbar sind und als Lackfilm einen hohen Vernetzungsgrad aufweisen.

Die Formulierungen der Lacke für die verschiedenen Einsatzzwecke, sowie die Herstellung und Verarbeitung solcher Lacke, sind dem Fachmann bekannt und der Fachliteratur zu entnehmen.

Die Beispiele erläutern die Erfindung, ohne sie in ihrem Umfang zu beschränken. Alle Angaben in Teilen oder Prozenten beziehen sich, soferne nichts anderes angegeben ist, auf Gewichtseinheiten.

In den Beispielen werden folgende Abkürzungen für Rohstoffe verwendet:

a) Diisocyanate:

HDI      Hexamethylendiisocyanat
IPDI     Isophorondiisocyanat

b) (Meth)acryloylgruppen enthaltende Alkohole:

ALK 1    2-Hydroxy-1-acryloxy-3-methacryloxypropan (Umsetzungsprodukt von Glycidylmethacrylat mit Acrylsäure)
ALK 2    Polypropylenglykolmonoacrylat (Bisomer PPA6E; Fa. INSPEC, GB)
ALK 3    Hexandioldiglycidylether-diacrylat
ALK 4    Hydroxyethylacrylat
ALK 5    Hydroxypropylacrylat

ALK 6    Umsetzungsprodukt von TMP-3EO (Polyol TP 30®, Fa. PERSTORP, SE) mit 2 Mol Acrylsäure, MG 373, OH-Zahl: 150 mg KOH/g,

ALK 7    Pentaerythrittriacrylat

c) aliphatische Monoalkohole:

ALK 8    2-Ethylhexanol
ALK 9    Isononylalkohol

d) DMPS Dimethylolpropionsäure

e) N,N-Dialkylalkanolamine:

DMEA   N,N -Dimethylethanolamin
DEEA   N,N -Diethylethanolamin

f) Katalysatoren:

TEA    Triethylamin
DBTL   Dibutylzinndilaurat

g) Polymerisationsinhibitoren:

HMME   Hydrochinonmonomethylether
HQ     Hydrochinon
BHT    Butyliertes Hydroxytoluol

1. Herstellung der Komponenten (A1) bis (A4)

Gemäß den Angaben in Tabelle 1 wird 1,0 Mol HDI in Gegenwart eines geeigneten Katalysators (DBTL) durch Zutropfen von 1,0 Mol Alkohol während 30 Minuten bis 3 Stunden bei max. 40°C zum entsprechenden Monourethan umgesetzt. Die Reaktionstemperatur ist abhängig von der Art und Qualität der verwendeten Alkohole (primäre oder sekundäre Hydroxylgruppen, Verunreinigungen) und sie muß für die einzelnen Ausgangsprodukte in Vorversuchen ermittelt werden. Das Reaktionsgemisch wird so lange bei der entsprechenden Temperatur gehalten, bis der theoretische NCO-Gehalt erreicht worden ist.

2. Herstellung der Komponenten (C1) bis (C5)

Analog zu Abschnitt 1, die Reaktionstemperatur für IPDI beträgt max. 55°C.

3. Herstellung der Komponenten (E1) bis (E3)

Gemäß den Angaben in Tabelle 2 werden unter den Komponenten (A) und (C) ausgewählte Produkte nach Zusatz eines Polymerisationsinhibitors (HQ, HMME) bei max. 50°C in einer stark exothermen Reaktion mit einem N,N -Dialkylalkanolamin umgesetzt, bis keine freien NCO-Gruppen nachweisbar sind, wobei das N,N - Dialkylalkanolamin vorgelegt und Luft in den Reaktionsansatz eingeleitet wird.

Tabelle 1

| | Tle (Mol) Diisocyanat | | | Tle (Mol) Alkohol | | | Reaktionstemperatur | MG | NCO-Gehalt in Gew.-% | DBÄ (Mmol/g) |
|---|---|---|---|---|---|---|---|---|---|---|
| (A1) | 168 | (1,0) | HDI | 183 | (0,85) | ALK 1 | 30 - 40°C | 372,6 | 11,2 | 4,56 |
| | | | | 21,6 | (0,15) | ALK 9 | | | | |

Tabelle 1 (fortgesetzt)

| | Tle (Mol) Diisocyanat | | | Tle (Mol) Alkohol | | | Reaktionstemperatur | MG | NCO-Gehalt in Gew.-% | DBÄ (Mmol/g) |
|---|---|---|---|---|---|---|---|---|---|---|
| (A2) | 168 | (1,0) | HDI | 47 | (0,2) | ALK 3 | 30 - 40°C | 319 | 13,1 | 3,13 |
| | | | | 104 | (0,8) | ALK 5 | | | | |
| (A3) | 168 | (1,0) | HDI | 252 | (0,6) | ALK 2 | 30 - 40°C | 539 | 7,7 | 3,34 |
| | | | | 119 | (0,4) | ALK 7 | | | | |
| (A4) | 168 | (1,0) | HDI | 64,5 | (0,3) | ALK 1 | 30 - 40°C | 445 | 9,4 | 3,60 |
| | | | | 186,5 | (0,5) | ALK 6 | | | | |
| | | | | 26 | (0,2) | ALK 8 | | | | |
| (C1) | 134,4 | (0,8) | HDI | 168 | (0,4) | ALK 2 | 50°C | 424,8 | 9,8 | 1,41 |
| | 44,4 | (0,2) | IPDI | 26 | (0,2) | ALK 5 | | | | |
| | | | | 52 | (0,4) | ALK 8 | | | | |
| (C2) | 222 | (1,0) | IPDI | 116 | (1,0) | ALK 4 | 50°C | 338 | 12,4 | 2,95 |
| (C3) | 168 | (1,0) | HDI | 26 | (0,2) | ALK 5 | 30 - 40°C | 305 | 13,7 | 0,66 |
| | | | | 39 | (0,3) | ALK 8 | | | | |
| | | | | 72 | (0,5) | ALK 9 | | | | |
| (C4) | 222 | (1,0) | IPDI | 23,2 | (0,2) | ALK 4 | 50°C | 349,2 | 12,0 | 0,57 |
| | | | | 104 | (0,8) | ALK 8 | | | | |
| (C5) | 168 | (1,0) | HDI | 130 | (1,0) | ALK 5 | 30 - 40°C | 298 | 14,0 | 3,35 |

Tabelle 2

| | Tle (Mol) Diisocyanat-VP | | | Alkanolamin | Tle (Mol) | | Reaktionstemperatur | MG | DBÄ |
|---|---|---|---|---|---|---|---|---|---|
| (E1) | 298 | (1,0) | (C5) | 89 | (1,0) | DMEA | 50°C | 387 | 2,58 |
| (E2) | 338 | (1,0) | (C2) | 89 | (1,0) | DMEA | 50°C | 427 | 2,33 |
| (E3) | 338 | (1,0) | (C2) | 118 | (1,0) | DEEA | 50°C | 456 | 2,18 |

### 4. Beispiele 1 bis 4

Gemäß den Angaben in Tabelle 3 werden zur Komponente (A) ein Polymerisationsinhibitor (HQ, HMME, BHT) und DMPS zugegeben. Der Reaktionsansatz wird unter Beachtung der exothermen Reaktion bei 70°C gehalten, wobei ständig Luft durch die Masse geleitet wird. Bei Bedarf kann die Temperatur allmählich auf 90°C gesteigert werden. Das Ende der Reaktion ist bei jenem NCO-Gehalt erreicht, der dem vollständigen Umsatz aller Hydroxylgruppen entspricht.

Nach Zugabe der Komponente (C) und gegebenenfalls eines Katalysators (DBTL, TEA) wird der Reaktionsansatz auf max. 110°C erwärmt und bei dieser Temperatur gehalten, bis keine freien NCO-Gruppen nachweisbar sind.

Das Reaktionsprodukt wird abschließend gemäß den Angaben in Tabelle 3 neutralisiert und mit deionisiertem Wasser auf den gewünschten Festkörpergehalt verdünnt.

### 5. Vergleichsbeispiele V1 und V2

Gemäß den Angaben in EP 0453838 A2, Beispiel 5 bzw. 6 hergestellte Produkte waren in Stufe c) bei einem Gehalt an noch nicht umgesetzten NCO-Gruppen von 1 % bzw. 2,5 % bei 100°C bereits so zähflüssig, daß sie mit Butylacetat verdünnt werden mußten.

Tabelle 3

| Komponente | Beispiel 1 Tle (Mol) | Beispiel 2 Tle (Mol) | Beispiel 3 Tle (Mol) | Beispiel 4 Tle (Mol) |
|---|---|---|---|---|
| (A1) | 372,6 (1,0) | | | |
| (A2) | | 319 (1,0) | | |
| (A3) | | | 539 (1,0) | |
| (A4) | | | | 445 (1,0) |
| (B) | 60,3 (0,45) | 53,6 (0,40) | 46,9 (0,35) | 37,5 (0,28) |
| (C1) | 127,5 (0,3) | | | |
| (C2) | | 118,3 (0,35) | | |
| (C3) | | | 122 (0,40) | |
| (C4) | | | | 87,3 (0,25) |
| NCO : OH in Äquiv. | 1,18 : 1 | 1,25 : 1 | 1,33 : 1 | 1,38 : 1 |
| Säurezahl | 45 | 46 | 28 | 28,5 |
| (D) LiOH | 5,19 g | 4,84 g | 5,73 g | 3,48 g |
| (E1) | 20,9 g | | | |
| (E2) | | | 34,0 g | |
| (E3) | | | | 66,1 g |
| Neutralisa-tionsgrad | 60 % | 50 % | 90 % | 100 % |
| D : E in Äquiv.-% | 80 : 20 | 100 : 0 | 75 : 25 | 50 : 50 |
| DBÄ | 3,30 Mmol/g | 2,72 Mmol/g | 2,62 Mmol/g | 2,81 Mmol/g |
| aliphatische Monoalkohole | 12 Mol-% | 0 Mol-% | 15 Mol-% | 22 Mol-% |

6. Lacktechnische Prüfung der gemäß den Beispielen 1 bis 4 hergestellten wasserverdünnbaren Urethanharze

Wasserverdünnbarer, UV-härtbarer Weißlack

| | |
|---|---|
| 250,0 | Urethanharz, 40 % in Wasser, gemäß den Beispielen 1 oder 2 |
| 80,0 | TiO$_2$ (Rutil) |
| 0,9 | Entschäumer, silikonfrei |
| 0,4 | Verlaufsmittel, silikonfrei |
| 2,0 | Photoinitiator 1 (DAROCUR® 1173,CIBA-GEIGY) |
| 1,0 | Photoinitiator 2 (LUCIRIN® TPO, BASF) |
| 30,0 | Wasser, deionisiert |
| $\overline{364,3}$ | |

Wasserverdünnbarer, UV-härtbarer Klarlack

| | |
|---|---|
| 100,0 | Urethanharz, 40%ig in Wasser gemäß den Beispielen 3 oder 4 |
| 0,5 | Entschäumer, silikonfrei |
| 1,0 | Untergrundbenetzungsmittel |
| 2,0 | Photoinitiator (DAROCUR® 1173, CIBA-GEIGY) |
| 5,0 | Wasser, deionisiert |
| $\overline{108,5}$ | |

Die in üblicher Weise hergestellten Lacke werden mit LiOH, 5%ig in Wasser, auf einen pH-Wert von 7,9 bis 8,3 eingestellt, mit deionisiertem Wasser auf die erforderliche Viskosität verdünnt und durch Spritzapplikation auf Glas- oder Blechplatten aufgebracht.

Härtungsbedingungen:

Forcierte Lufttrocknung während 5 min bei 55°C, UV-Härtung mit Quecksilber-Hochdruck-Lampen (pigmentierte Formulierung: Gallium-dotierte QuecksilberHochdrucklampen), 80 Watt/cm, Objektabstand ca. 10 cm, Bandgeschwindigkeit 4 m/min.

Prüfmethoden und Prüfergebnisse:

Pendelhärte:

gemäß DIN 53157, Glasplatten, Schichtstärke (Naßfilm) 150 μm.

Mechanische Verformbarkeit (Erichsentiefung):

Prüfung gemäß ISO 1520, Bonderblech, Schichtstärke (Naßfilm) 120 μm.

Acetonfestigkeit:

gemäß DIN 68861, Glasplatten, Schichtstärke (Naßfilm) 150 μm.

|  | Weißlack | | Klarlack | |
|---|---|---|---|---|
| Bindemittel | Beispiel 1 | Beispiel 2 | Beispiel 3 | Beispiel 4 |
| Pendelhärte [s] | 168 | 156 | 170 | 162 |
| Tiefung [mm] | 7,8 | 7,4 | 8,8 | 8,7 |
| Acetonfestigkeit [min] | 150 | 135 | 188 | 200 |

**Patentansprüche**

1. Verfahren zur Herstellung von wasserverdünnbaren Urethanharzen, <u>dadurch gekennzeichnet</u>, daß man

(A) 1,0 Mol      Hexamethylendiisocyanat, dessen NCO-Gruppen nach der Reaktion mit (Meth)acryloylgruppen enthaltenden Alkoholen, welche als eine oder zwei freie Hydroxylgruppe(n) aufweisende Ester der (Meth)acrylsäure mit zwei- bis vierwertigen Alkoholen bzw. mit Mono- oder Diglycidylverbindungen zu definieren sind, und gegebenenfalls mit aliphatischen Monoalkoholen, deren Alkylreste sechs bis vierzehn C-Atome aufweisen, zu insgesamt 50 Mol-% als Urethangruppen vorliegen, mit

(B) 0,25 bis 0,45 Mol      2,2-Bis-(hydroxymethyl)-propionsäure bei 70 bis 90°C bis zur vollständigen Reaktion der Hydroxylgruppen umsetzt, anschließend das so erhaltene Zwischenprodukt (AB) mit

(C) 0,2 bis 0,45 Mo      l eines aliphatischen und/oder cycloaliphatischen Diisocyanats, dessen NCO-Gruppen nach der Reaktion mit (Meth)acryloylgruppen enthaltenden Alkoholen, welche als eine einzige freie Hydroxylgruppe aufweisende Ester der (Meth)acrylsäure mit zwei- bis vierwertigen Alkoholen bzw. mit Mono- oder Diglycidylverbindungen zu definieren sind, und gegebenenfalls mit aliphatischen Monoalkoholen, deren Alkylreste sechs bis vierzehn C-Atome aufweisen, zu insgesamt 50 Mol-% als Urethangruppen vorliegen,

bei 100 bis 110°C bis zur vollständigen Reaktion der restlichen freien Isocyanatgruppen zu Allophanatgruppen umsetzt, wobei die Molverhältnisse der Komponenten (A) bis (C) so gewählt werden, daß sich die Äquivalente der in den ursprünglichen Rohstoffen für die Komponenten (A) bis (C) enthaltenen Isocyanatgruppen und Hydroxylgruppen wie 1,1 : 1 bis 1,45 : 1 verhalten und das Reaktionsprodukt Carboxylgruppen entsprechend einer Säurezahl von 25 bis 50 mg KOH/g aufweist, die zu mindestens 45 % mit einem (D) Alkalihydroxid, gegebenenfalls als Gemisch mit einem (E) aliphatischen oder cycloaliphatischen Diisocyanat, dessen NCO-Gruppen nach der Reaktion zu je 50 Mol-% mit (Meth)acryloylgruppen enthaltenden Alkoholen, welche wie bei Komponente (C) als eine einzige freie Hydroxylgruppe aufweisende Ester der (Meth)acrylsäure mit zwei- bis vierwertigen Alkoholen bzw. mit Mono- oder Diglycidylverbindungen zu definieren sind, sowie mit N,N-Dialkylalkanolaminen als Urethangruppen vorliegen, neutralisiert werden, mit der Maßgabe, daß das Endprodukt ein Doppelbindungsäquivalent (Mol-Zahl der ethylenischen Doppelbindungen pro 1000 g Harz als Feststoff) von 1,5 bis 3,5 mMol/g aufweist.

2. Verfahren gemäß Anspruch 1, dadurch gekennzeichnet, daß man als aliphatisches Diisocyanat in den Komponenten (C) und (E) Hexamethylendiisocyanat einsetzt.

3. Verfahren gemäß Anspruch 1, dadurch gekennzeichnet, daß man als cycloaliphatisches Diisocyanat in den Komponenten (C) und (E) Isophorondiisocyanat einsetzt.

4. Verfahren gemäß Anspruch 1, dadurch gekennzeichnet, daß man die aliphatischen Monoalkohole in den Komponenten (A) und (C) in einem Anteil von höchstens 40 Mol-%, bezogen auf die gesamte Menge an Hydroxylgruppen, die zur Blockierung von NCO-Gruppen zur Verfügung stehen, einsetzt.

5. Verfahren gemäß Anspruch 1, dadurch gekennzeichnet, daß man als Komponente (D) Lithiumhydroxid einsetzt.

6. Verfahren gemäß Anspruch 1, dadurch gekennzeichnet, daß man als N,N-Dialkylalkanolamin in Komponente (E) N,N-Dimethylethanolamin oder N,N-Diethylethanolamin einsetzt.

7. Verfahren gemäß Anspruch 1, dadurch gekennzeichnet, daß man in einem Gemisch der Komponenten (D) und (E) die Komponente (E) in einem Anteil bis zu 50 Äquivalent-% einsetzt.

8. Verwendung der gemäß den Ansprüchen 1 bis 7 hergestellten wasserverdünnbaren Urethanharze als Bindemittel für durch radikalische Polymerisation, insbesonders mittels UV-Bestrahlung, vernetzbare Lacke, die in der Anwendungsform frei von Monomeren und organischen Lösemitteln vorliegen, emissionsfrei härtbar sind und als Lackfilm einen hohen Vernetzungsgrad aufweisen.

## Claims

1. Process for the preparation of water-dilutable urethane resins, characterized in that

| | |
|---|---|
| (A) 1.0 mol | of hexamethylene diisocyanate, a total of 50 mol% of whose NCO groups are in the form of urethane groups after the reaction with alcohols containing (meth)acryloyl groups, which are to be defined as esters of (meth)acrylic acid with di- to tetrahydric alcohols and/or with mono- or diglycidyl compounds, which esters have one or two free hydroxyl group(s), and, if desired, with aliphatic monoalcohols whose alkyl radicals have six to fourteen C atoms, is reacted with |
| (B) from 0.25 to 0.45 mol | of 2,2-bis(hydroxymethyl)propionic acid at from 70 to 90°C until complete reaction of the hydroxyl groups has taken place, and then the intermediate (AB) thus obtained is reacted with |
| (C) from 0.2 to 0.45 mol | of an aliphatic and/or cycloaliphatic diisocyanate, a total of 50 mol% of whose NCO groups are in the form of urethane groups after the reaction with alcohols containing (meth)acryloyl groups, which are to be defined as esters of (meth)acrylic acid with di- to tetrahydric alcohols and/or with mono- or diglycidyl compounds, which esters have one single free hydroxyl group and, if desired, with aliphatic monoalcohols, whose alkyl radicals have six to fourteen C atoms, |

at from 100 to 110°C until complete reaction of the remaining free isocyanate groups to give allophanate groups has taken place, the molar ratios of components (A) to (C) being chosen such that the ratio of equivalents of the isocyanate groups and hydroxyl groups present in the original raw materials for components (A) to (C) is from 1.1:1 to 1.45:1 and the reaction product contains carboxyl groups corresponding to an acid number of from 25 to 50 mg of KOH/g, at least 45% of which are neutralized with (D) an alkali metal hydroxide, if desired as a mixture with (E) an aliphatic or cycloaliphatic diisocyanate whose NCO groups, after the reaction to the extent of 50 mol% each with alcohols containing (meth)acryloyl groups, which are to be defined, as in the case of component (C), as esters of (meth)acrylic acid with di- to tetrahydric alcohols and/or with mono- or diglycidyl compounds, which esters have one single free hydroxyl group, and with N,N-dialkylalkanolamines, are in the form of urethane groups, with the proviso that the end product has a double bond equivalent (number of moles of ethylenic double bonds per 1000 g of resin as solid) of from 1.5 to 3.5 mmol/g.

2. Process according to Claim 1, characterized in that the aliphatic diisocyanate employed in components (C) and (E) is hexamethylene diisocyanate.

3. Process according to Claim 1, characterized in that the cycloaliphatic diisocyanate employed in components (C) and (E) is isophorone diisocyanate.

4. Process according to Claim 1, characterized in that the aliphatic monoalcohols in components (A) and (C) are employed in a proportion of not more 40 mol%, based on the overall quantity of hydroxyl groups available for blocking NCO groups.

5. Process according to Claim 1, characterized in that lithium hydroxide is employed as component (D).

6. Process according to Claim 1, characterized in that, as N,N-dialkylalkanolamine in component (E), N,N-dimethylethanolamine or N,N-diethylethanolamine is employed.

7. Process according to Claim 1, characterized in that in a mixture of components (D) and (E) component (E) is employed in a proportion of up to 50 equivalent %.

8. Use of the water-dilutable urethane resins prepared according to Claims 1 to 7 as binders for coating materials which can be crosslinked by free-radical polymerization, especially by means of UV radiation, and which, in the form in which they are employed are free from monomers and organic solvents, can be cured without emissions and, as coating film, show a high degree of crosslinking.

## Revendications

1. Procédé de préparation de résines d'uréthane diluables à l'eau, caractérisé en ce que l'on fait réagir

(A) 1,0 mole — d'hexaméthylènediisocyanate dont les groupes NCO sont présents à raison de 50 mol% au total sous forme de groupes uréthane après la réaction avec des alcools contenant des groupes (méth)acryloyle qui sont à définir comme des esters de l'acide (méth)acrylique comportant un ou deux groupes hydroxyle libres avec des alcools di- à tétrafonctionnels ou avec des composés mono- ou diglycidyliques, et éventuellement avec des monoalcools aliphatiques dont les restes alkyle comportent six à quatorze atomes de carbone,
avec

(B) 0,25 à 0,45 mole — d'acide 2,2-bis-(hydroxyméthyl)propionique entre 70 et 90°C jusqu'à la réaction totale des groupes hydroxyle,
puis on fait réagir le produit intermédiaire (AB) ainsi obtenu avec

(C) 0,2 à 0,45 mole — d'un diisocyanate aliphatique et/ou cycloaliphatique dont les groupes NCO sont présents à raison de 50 mol% au total sous forme de groupes uréthane après la réaction avec des alcools contenant des groupes (méth)acryloyle qui sont à définir comme les esters de l'acide (méth)acrylique comportant un seul groupe hydroxyle libre avec des alcools di- à tétrafonctionnels ou avec des composés mono- ou diglycidyliques, et éventuellement avec des monoalcools aliphatiques dont les restes alkyle comportent six à quatorze atomes de carbone,

entre 100 et 110°C jusqu'à la réaction totale des groupes isocyanates libres restants en groupes allophanate, les proportions molaires des composants (A) à (C) étant choisies de telle manière que les équivalents des groupes isocyanate et des groupes hydroxyle contenus dans les matières premières initiales pour les composants (A) à (C) sont entre eux dans le rapport 1,1:1 à 1,45:1 et que le produit de la réaction comporte des groupes carboxyle d'une manière correspondant à un indice d'acide de 25 à 50 mg de KOH/g, qui sont neutralisés à raison d'au moins 45% avec un hydroxyde alcalin (D), éventuellement sous forme de mélange avec un diisocyanate aliphatique ou cycloaliphatique (E) dont les groupes NCO après la réaction à raison de respectivement 50 mol% avec des alcools contenant des groupes (méth)acryloyle, qui, comme dans le cas du composant (C), sont à définir comme des esters de l'acide (méth)acrylique comportant un seul groupe hydroxyle libre avec des alcools di- à tétrafonctionnels ou avec des composés mono- ou diglycidyliques, ainsi qu'avec des N,N-dialkylalcanolamines sont sous forme de groupes uréthane, à condition que le produit final présente un équivalent de doubles liaisons (nombre de moles des doubles liaisons éthyléniques pour 1000 g de résine sous forme de solide) de 1,5 à 3,5 mmol/g.

2. Procédé selon la revendication 1 caractérisé en ce que l'on utilise comme diisocyanate aliphatique dans les composants (C) et (E) l'hexaméthylènediisocyanate.

3. Procédé selon la revendication 1 caractérisé en ce que l'on utilise comme diisocyanate cycloaliphatique dans les composants (C) et (E) l'isophoronediisocyanate.

4. Procédé selon la revendication 1 caractérisé en ce que l'on utilise les monoalcools aliphatiques dans les composants (A) et (C) en une proportion d'au plus 40 mol% par rapport à la quantité totale de groupes hydroxyle qui sont disponibles pour le blocage des groupes NCO.

5. Procédé selon la revendication 1 caractérisé en ce que l'on utilise l'hydroxyde de lithium comme composant (D).

6. Procédé selon la revendication 1 caractérisé en ce que l'on utilise la N,N-diméthyléthanolamine ou la N,N-diéthyléthanolamine comme N,N-dialkylalcanolamine dans le composant (E).

7. Procédé selon la revendication 1 caractérisé en ce que l'on utilise le composant (E) en une proportion de jusqu'à 50 équivalent% dans un mélange des composants (D) et (E).

8. Utilisation des résines d'uréthane diluables à l'eau préparées selon les revendications 1 à 7 comme liant pour des peintures réticulables par polymérisation radicalaire, en particulier au moyen d'une irradiation UV, qui, dans la forme pour l'utilisation, sont exemptes de monomères et de solvants organiques, durcissables sans émissions et présentent sous forme de film de peinture un haut degré de réticulation.